# EUROPEAN PATENT SPECIFICATION

(11) **EP 0 384 766 B1**
(45) Date of publication and mention of the grant of the patent: **06.07.1994**
(21) Application number: 90301933.9
(22) Date of filing: 22.02.1990
(51) Int. Cl.: C07C 209/16, C07D 295/12

(54) **Process of preparing linearly-extended polyalkylenepolyamines**
Verfahren zur Herstellung von linear ausgebreiteten Polyalkylenpolyaminen
Procédé de préparation de polyalkylènepolyamines étendues linéairement

(30) Priority: 23.02.1989 US 314528
(43) Date of publication of application: 29.08.1990
(73) Proprietor: THE DOW CHEMICAL COMPANY, Midland, Michigan 48640 (US)
(72) Inventor: Bowman, Robert G., Midland, Michigan 48640 (US); Hartwell, George E., Midland, Michigan 48640 (US); Molzahn, David C., Midland, Michigan 48640 (US); Ramirez, Enrique G., Lake Jackson, Texas 77566 (US); Lastovica, Jr. John E., Lake Jackson, Texas 77566 (US)
(74) Representative: Burford, Anthony Frederick

(56) References cited:
- EP-A- 0 228 898
- EP-A- 0 256 516
- EP-A- 0 328 101
- GB-A- 2 147 896
- US-A- 2 394 515
- PATENT ABSTRACTS OF JAPAN, vol. 13, no. 151 (C-584)[3499], 12th April 1989; & JP-A-63 307 846
- PATENT ABSTRACTS OF JAPAN, vol. 11, no. 3 (C-395)[2450], 7th January 1987; & JP-A-61 183 249

## Description

This invention relates to a process for preparing linearly-extended polyalkylenepolyamines, such as diethylenetriamine, and linear and branched striethylenetetramines Linearly-extended polyalkylenepolyamines also include alcohol-extended piperazines, such as N-(2-hydroxyethyl)piperazine, and amine-extended piperazines, such as N-(2-aminoethyl)piperazine.

Linearly-extended polyalkylenepolyamines find utility as dispersants, surfactants, chelants, catalysts, curing agents, extenders in polyurethanes, and as starting materials or intermediates in the preparation of pesticides, veterinary antihelmintic pharmaceuticals, and high temperature lubricating oils.

It is known that non-cyclic polyalkylenepolyamines can be prepared by the reaction of an alkyl halide with ammonia or an amine. The product is a polyalkylenepolyamine hydrohalide salt, which must be neutralized with base in order to recover the valuable polyalkylenepolyamine product. The neutralization produces a waste stream of metal salt, which must be removed. Moreover, the process produces a considerable amount of cyclic compounds.

It is known that salt-free linear polyethylenepolyamines can be directly prepared by reacting an ethyleneamine with an ethanolamine in the presence of hydrogen and a hydrogenation catalyst. For example, US-A-3,714,259 discloses such a process with preferred catalysts derived from the oxides of chromium, copper, nickel, and cobalt.

More recently it is known to prepare non-cyclic polyalkylenepolyamines directly by reacting an alkanolamine with an alkyleneamine under non-reductive conditions, that is, in the absence of hydrogen. Many of the non-reductive aminations are known to employ a phosphorous containing catalyst. US-A-4,036,881, for example, teaches the use of acidic metal phosphates, phosphoric acid compounds, and organic phosphates in the reaction of an alkanolamine with an alkyleneamine. US-A-4,448,997 teaches a similar process except that an aluminum phosphate catalyst prepared from alumina and phosphoric acid is employed. These phosphorus-containing catalysts are soluble in amines. Consequently, the catalysts leach into the reaction causing catalyst losses and separation problems.

Other non-reductive processes are known employing phosphorus-containing catalysts. US-A-4,463,193, for example, discloses the production of non-cyclic polyalkylenepolyamines by reacting an alkanolamine with an alkyleneamine and ammonia in the presence of a Group IIIB metal acid phosphate, including the rare earth lanthanide metals. US-A-4,578,518 is representative of a series of patents drawn to the production of linear polyethylenepolyamines from ethylenediamine and monoethanolamine using catalysts comprising titania having phosphorus deposited thereon. These phosphorus-containing catalysts lose their physical integrity in the presence of water, which is a by-product of the amination reaction. Moreover, these catalysts can react with water to release free phosphoric acid or amine phosphate salts. Consequently, these catalysts can lose their phosphorus component and leach into the reaction mixture causing catalyst losses and separation problems.

UK-B-2,147,896 discloses a process for producing diethylenetriamine by reacting monoethanol-amine with ammonia in the presence of ethylenediamine and a Group VB metal phosphate. The mole ratio of phosphorus to Group VB metal is disclosed to be 1 for vanadium, and 1.5 and 3 for niobium and tantalum. Disadvantageously, these catalysts decompose in the reaction mixture, thereby shortening the catalyst lifetime and causing problems similar to those mentioned hereinbefore.

EP-A-0256516 discloses a process for preparing alkyleneamine by using a niobium-containing substance, especially niobium pentoxide or a niobate, as a catalyst. This patent also discloses that phosphorus-type supported solid catalysts are liable to separate from the surface of the carrier when the active sites of the catalysts are exposed to high temperatures in the presence of water, resulting in reduced activity of the catalysts with the lapse of time.

US-A-3,364,218 teaches the self-condensation of N-(2-hydroxyethyl)piperazine to poly-1,4-ethylene-piperazine in the presence of hydrogen and a solid acid catalyst, such as silica-alumina, alumina, tungsten oxide, aluminum phosphate, and acid clays. It is difficult to control the degree of polymerization in this process. Accordingly, it is difficult to obtain high yields of N-(2-aminoethyl)piperazine, 1,2-bis-(piperazinyl)ethane, tris(piperazinylethane), or other lower oligo(piperazinylethanes). Moreover, cyclic compounds, such as 1,4-diaza-[2.2.2]-bicyclooctane, are produced as undesirable by-products. In addition, the catalysts employed in this process lose their physical integrity in the presence of amines and water; therefore, the process is hampered by catalyst losses and separation problems.

US-A-4,552,961 discloses a process for the preparation of polyalkylene polypiperazines comprising reacting piperazine with alkylene glycols or alkanolamines in the presence of a catalyst of phosphorus amide. Disadvantageously, this catalyst is homogeneous and must be separated from the product stream.

US-A-2394515 discloses the preparation of poly-alkyl amines by alkylation of ammonia with an alkanol in the presence of a catalyst comprising an aluminum oxide or salt, silica and vanadium oxide.

JP-A-63307846 discloses the preparation of alkyleneamines by heating ammonia or an alkyleneamine with an alkanolamine in the presence of a tantalum-containing compound, especially tantalum pentoxide or a tantalate.

EP-A-0328101 (published 16th August 1989) discloses the preparation of alkyleneamines by heating ammonia or an alkyleneamine with an alkanolamine in the presence of a supported niobium-containing compound, especially niobium pentoxide or a niobate.

It would be advantageous to have a process for the direct amination of aliphatic alcohols to polyalkylenepolyamines which does not require large amounts of hydrogen and expensive metals. It would be more advantageous if such a process produces high selectivity for linearly-extended products and low selectivity for undesirable cyclic materials. It would be more advantageous if the catalyst for such a process is insoluble in the liquid reactant amines. It would be most advantageous if the catalyst for such a process retains its physical integrity in the presence of water. Such a process would eliminate the need for neutralizing hydrohalide salts and disposing of a waste salt stream. Such a process would also eliminate problems with catalyst leaching, reactor plugging, and catalyst separation. Accordingly, such an amination process would be suitable for industrial purposes.

In one aspect, this invention is a process for preparing linearly-extended polyalkylenepolyamines which comprises contacting a difunctional aliphatic alcohol with a reactant amine in the presence of a catalyst containing a niobium phosphate or tantalum phosphate wherein the mole ratio of phosphorus to niobium or tantalum metal is no greater than 1.35. The contacting of the difunctional aliphatic alcohol and the reactant amine with the catalyst is conducted under reaction conditions such that a mixture of polyalkylenepolyamines enriched in linearly-extended products is produced. For the purposes of this invention "linearly-extended products" are defined as amine products arising from the condensation of the difunctional aliphatic alcohol and amine reactants. Non-cyclic products include linear and branched homologues. Linearly-extended products are to be distinguished from cyclic products, which arise from condensation of the alcohol and amine reactants followed by internal cyclization to form a nitrogen-containing heterocycle.

Advantageously, the process of this invention is capable of achieving higher yields of the valuable linearly-extended polyalkylenepolyamines, and lower yields of undesirable cyclic products. More advantageously, the process of this invention does not produce a waste stream of metal salts. Even more advantageously, the catalysts of this invention are insoluble in liquid amines; therefore, catalyst losses are minimized and separation of the polyamine products from the catalyst is relatively easy. Most advantageously the catalysts of this invention retain their physical integrity in the presence of water. Consequently, the catalysts possess a long lifetime and are suitable for industrial use.

The linearly-extended polyalkylenepolyamine products of this invention are useful as dispersants, surfactants, chelants, curing agents, and catalysts, and also useful in the formation of ureas, urethane polymers, and pesticides.

The difunctional aliphatic alcohol which is employed in the process of this invention includes any aliphatic alcohol containing (a) at least one hydroxyl moiety bound to a primary carbon atom, and (b) at least one additional moiety selected from the group consisting of hydroxyl, primary amine or secondary amine functionalities. Examples of suitable difunctional aliphatic alcohols include diols such as ethylene glycol and propylene glycol, triols such as glycerol, and higher polyols; polyether polyols such as diethylene glycol, ethylene oxide capped polypropylene glycols, and higher homologues; alkanolamines such as monoethanolamine and N-(2-aminoethyl)ethanolamine; and polyether amino alcohols such as 2-(β-aminoethoxy)ethanol; and hydroxyalkyl-substituted piperazines, such as N-(2-hydroxyethyl)piperazine, N,N'-bis(2-hydroxyethyl)piperazine, and N-(2-hydroxyethyl)bispiperazine. The difunctional alcohols are not limited to the aforementioned examples, and other equally suitable difunctional alcohols can be employed in the practice of this invention.

Preferably, the difunctional alcohols which are polyols, polyether amino alcohols, or alkanolamines are represented by the general formula I: 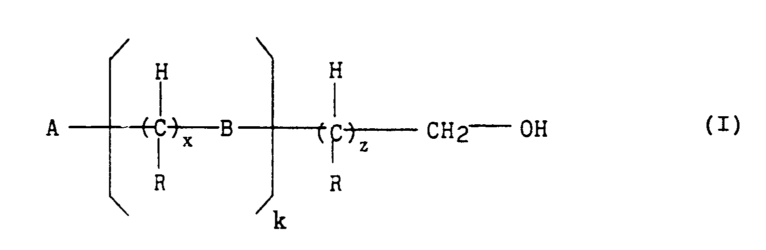
wherein A is OH or NHR'; each B is independently NR' or O; each R is independently hydrogen, hydroxyl, amino (NH₂), an alkyl moiety of 1-12 carbon atoms such as methyl, ethyl or propyl, a hydroxyalkyl or aminoalkyl moiety of 1-12 carbon atoms, or a monocyclic aromatic moiety, such as phenyl or tolyl; each R' is independently hydrogen, hydroxyl, an alkyl moiety of 1-12 carbon atoms such as methyl, ethyl or propyl, a hydroxyalkyl or aminoalkyl moiety of 1-12 carbon atoms, or a monocyclic aromatic moiety, such as phenyl or tolyl; x is a positive integer from 2 to 12; k is a positive integer from 0 to 150; and z is a positive integer from 0 to 12. Preferably, each R and R' is hydrogen. More preferably, each R and R' is hydrogen, x is 2, and z is 1. Most preferably, each R and R' is hydrogen, A is NH₂, k is 0, z is 1, and the difunctional aliphatic alcohol is monoethanolamine.

In those reactions wherein the difunctional alcohol contains a piperazine moiety, the preferred difunctional alcohols are represented by the general formula II: 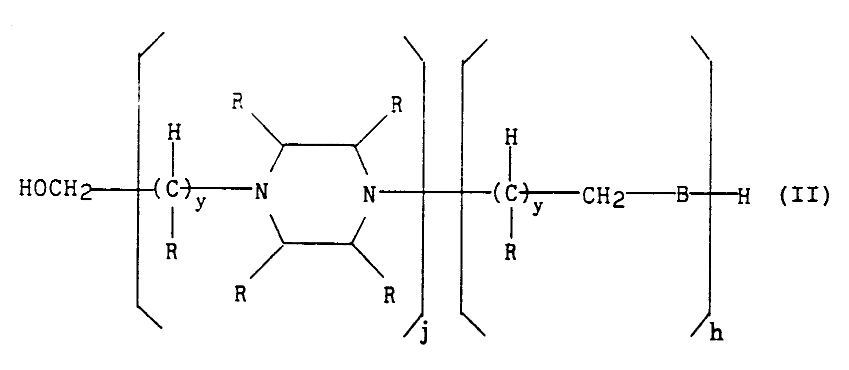
wherein each B, R and R' is as defined above in connection with formula I; each y is independently an integer from 0 to 12; j is an integer from 1 to about 6; and h is an integer from 0 to 6. Some examples of difunctional alcohols which satisfy this formula are N-(2-hydroxyethyl)piperazine, N-(2-hydroxyethyl)bispiperazine, N,N'-bis(2-hydroxyethyl)piperazine, and N,N' -bis(2-hydroxyethyl)bispiperazine. Preferably, each R and R' is hydrogen. More preferably, each R is hydrogen, each y is independently 1 or 2, j is 1 or 2, h is 0, 1, or 2, and B is NR'. Most preferably, each R is hydrogen, y is 1, j is 1, h is 0, and the compound is N-(2 -hydroxyethyl)piperazine.

The reactant amines which are employed in the process of this invention include ammonia and any primary or secondary aliphatic amine which is capable of aminating the difunctional aliphatic alcohol. Examples of suitable reactant amines include aliphatic monoamines such as ethylamine, propylamine, n-butylamine, hexylamine, octylamine, diethylamine, dipropylamine, dibutylamine, dihexylamine, dicyclohexylamine, dioctylamine, methylethylamine, and ethylpropylamine; linear and branched alkylene diamines and polyamines such as ethylenediamine, propylenediamine, diethylenetriamine, triethylenetetramines, and tetraethylenepentamines; alkylene ether polyamines such as 2 -(β-aminoethoxy)ethylamine; and mixtures of the above-identified amines. While the aforementioned amines are representative of those which are suitable for the process of this invention, other amines not recited herein may be equivalent and equally suitable.

Simple primary and secondary reactant amines which are preferred for the process of this invention are represented by the general formula R¹₂NH, wherein each R¹ is independently hydrogen or a C₁-C₁₂ alkyl moiety. Preferably, the reactant alkylenepolyamines and alkylene ether polyamines which are suitable in the process of this invention are represented by the general formula III: 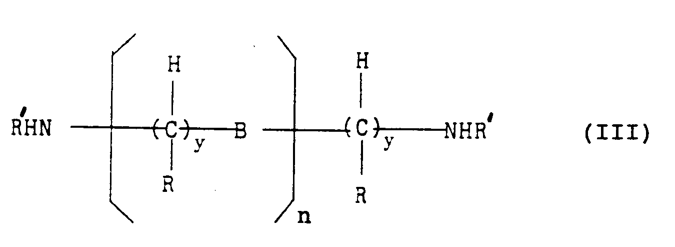
wherein each B, R and R' is as defined above in connection with formula I; each y is independently a positive integer from 2 to 12, and n is a positive integer from 0 to 150. Preferably, each B is NR' and the amine is an alkylenepolyamine. More preferably, the amine is an alkylenepolyamine and each R and R' is hydrogen. Even more preferably, each B is NR', each R is hydrogen, each y is 2, and the amine is an ethylenepolyamine. Most preferably, the reactant amine is ethylenediamine.

In those reactions wherein the reactant amine contains a piperazine moiety, preferred piperazines or aminoalkyl-substituted piperazines are represented by the general formula IV: 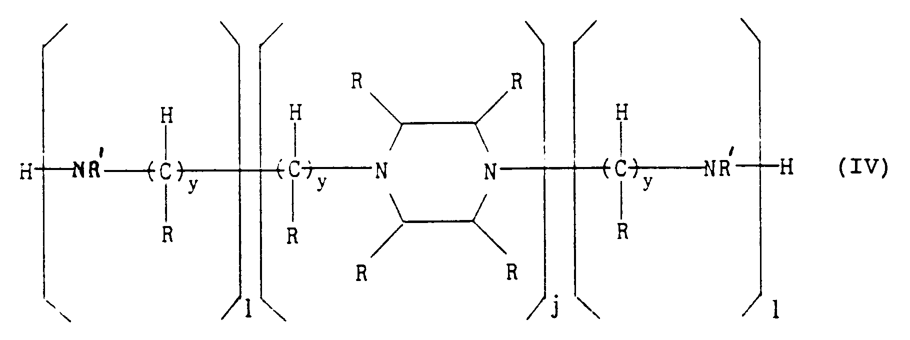
wherein each R and R' is as defined above in connection with formula I; each y is independently an integer from 0 to 12; each 1 is independently an integer from 0 to 6; and j is an integer from 1 to 6. Some examples of reactant amines which satisfy this formula include piperazine, N-(2-aminoethyl)piperazine, N,N'-bis-(2-aminoethyl)piperazine, bis(piperazinyl)ethane, and N-(2-aminoethyl)bispiperazine. Preferably, each R and R' is hydrogen. More preferably, each R and R' is hydrogen, y is 1 or 2, j is 1 or 2, and 1 is 0, 1, or 2. Most preferably, each R is hydrogen, y is 0, j is 1, and each 1 is 0, and the compound is piperazine.

In accordance with the process of this invention, any mole ratio of reactant amine to difunctional aliphatic alcohol which enables the amination reaction to proceed to the desired linearly-extended polyalkylenepolyamine products is suitable. Typically, the alcohol is reacted with at least one mole equivalent of reactant amine; however, an excess of reactant amine can be advantageously employed. Preferably, the mole ratio of reactant amine to difunctional aliphatic alcohol is in the range of from 0.1 to 20. More preferably, the mole ratio of reactant amine to difunctional aliphatic alcohol is in the range of from 1 to 15, even more preferably from 1 to 10, and most preferably from 2 to 10.

Although, preferably, a solvent is not used in the amination reaction, it is within the scope of the invention for a solvent to be used, if desired. Any solvent is acceptable provided that (1) it is not reactive with the difunctional alcohol and the reactant or product amines, and (2) it does not decompose under the conditions of the reaction. Some examples of suitable solvents include water, saturated aliphatic hydrocarbons such as pentane, hexane, heptane, octane, nonane, and decane, and aromatic hydrocarbons such as benzene, toluene, and xylene. The amount of solvent employed depends upon the particular reactants and reaction conditions. Any amount of solvent is acceptable that meets the intended purpose of use. If a solvent is used, the solvent typically constitutes from 5 weight percent to 95 weight percent of the feed stream. Preferably, the solvent constitutes from 10 weight percent to 80 weight percent of the feed stream.

The catalyst employed in the process of this invention is a composition containing a niobium phosphate or tantalum phosphate.

Examples of suitable niobium or tantalum phosphates include 2Nb₂O₅.P₂O₅.6H₂O, 2Nb₂O₅.P₂O₅, NbOPO₄, NbOPO₄.xH₂O, PNb₉O₂₅, 2Ta₂O₅.P₂O₅.6H₂O, 2Ta₂O₅.P₂O₅, TaOPO₄ and TaOPO₄.xH₂O. Niobium or tantalum meta-phosphates, fluorophosphates, hydrated phosphates, silicophosphates and non-stoichiometric phosphate compounds are also suitable, as are niobium or tantalum hydrogen phosphates. Preferably, the niobium or tantalum phosphate possesses a P/Nb or P/Ta mole ratio no greater than 1.3. More preferably, the niobium or tantalum phosphate possesses a P/Nb or P/Ta mole ratio in the range of from 0.01 to 1.3. Preferably, the phosphate is a niobium phosphate. More preferably, the phosphate is NbOPO₄ and the hydrated forms of NbOPO₄.

The niobium or tantalum phosphates are relatively easy to prepare. The preparations are also described in *Comprehensive Inorganic Chemistry*, ibid., pp. 612-613. Preferably, the niobium or tantalum phosphate is prepared by reacting a catalyst precursor compound containing niobium or tantalum with a phosphorus-containing compound, such as phosphoric acid, under conditions sufficient to generate the niobium or tantalum phosphate. Anhydrous or aqueous phosphoric acid can be employed, as can chlorinated or fluorinated phosphoric acids, or chlorinated or fluorinated phosphorus-containing compounds. Typical catalyst precursor compounds which can be employed as starting materials include niobium or tantalum oxides, hydrated metal oxides, halides, alkoxides, and carboxylic acid salts. Preferably, the precursor compound is a niobium or tantalum hydrated metal oxide. More specifically, the catalyst precursor is heated with phosphoric acid at about atmospheric pressure and at a temperature in the range from 130° to 200°C. The weight ratio of phosphoric acid to precursor compound is preferably in the range from 5 to 20, more preferably in the range from 7 to 15, most preferably, about 10. The phosphoric acid is typically employed as an 85 weight percent aqueous solution; however, additional water can be used to obtain phosphate catalysts having higher surface area. The heating time varies depending upon the quantity of precursor compound and the amount of water or other volatiles which are to be driven off in the heating. Typically, however, the mixture is heated for about one to two hours; however longer times may be employed. After heating, the mixture comprising a liquid phase and a solid phase is cooled. The liquid is decanted from the solid, and the solid is washed with water and filtered. The washing and filtering may be repeated several times to ensure the removal of excess acid and unwanted ions. The filtered solid is dried at a temperature in the range from 80°C to 150°C in air for a time in the range of from 2 hours to 50 hours to yield the phosphate catalyst of the invention. Typically, the catalyst is heat treated or calcined prior to use. Preferably, the calcination is conducted at a temperature in the range of from 200°C to 500°C for a time in the range of from 2 hours to 50 hours.

Preferably, the niobium phosphate and tantalum phosphate compounds, described hereinbefore, are insoluble in the amination reaction mixture, thereby acting as heterogeneous catalysts. Optionally, any of the metal phosphates can be made less soluble by (a) depositing onto a support material, or (b) binding with a metal oxide or support precursor. Any support or binder material is acceptable provided that it does not enhance the formation of undesirable cyclic products in the process of this invention. Suitable supports or binders include carbon and any refractory oxide such as alumina (hydrated and dehydrated forms), zirconia, boria, thoria, magnesia, titania, tantala, chromia, silica, kieselguhr, and mixtures of these materials. Preferably, the support or binder material is alumina, silica, or titania. More preferably, the support material or binder is an alumina or hydrated alumina, such as boehmite or pseudoboehmite alumina (aluminum oxyhydroxide). The support material typically has a surface area of at least 0.1 m²/g. Preferably, the support material has a surface area in the range from 5 m²/g to 600 m²/g, most preferably in the range from 50 m²/g to 200 m²/g. These surface areas are measured by the Brunauer-Emmett-Teller (BET) method, as described by R. B. Anderson, in Experimental Methods in Catalytic Research, Academic Press, 1968, pp 48-66.

The metal phosphates can be applied to the support material in any known fashion, such as by impregnation or by precipitation *in situ* from the catalyst preparation reaction. In these preparations the phosphate is adsorbed onto the support.

In a preferred method of preparation, the metal phosphate can be chemically reacted or bound onto the support. In this preparation a catalyst precursor compound, such as identified hereinbefore, is reacted with hydroxyl functionalities of the support to yield a catalyst precursor chemically bound to the support. For example, niobium chloride reacts with the hydroxyl moieties of silica to yield niobium chloride bound through an oxygen to silicon. Typically, the niobium chloride is dissolved in a solvent to make a solution. Any solvent is acceptable, provided that it is not reactive with the metal chloride or the supported metal chloride. Acceptable solvents include saturated hydrocarbons, such as pentane and hexane, and aromatic hydrocarbons, such as benzene and toluene, as well as acetone, acetonitrile and chlorinated hydrocarbons. Typically the minimum amount of solvent is used to dissolve the metal chloride. The refractory oxide is added to the resulting solution in a metal chloride/refractory oxide weight ratio in the range from 0.0005 to 0.60. The mixture is then rotary evaporated to remove the solvent leaving a solid of metal chloride supported on a refractory oxide. The solid is heated at a temperature in the range from 50°C to 150°C for a time in the range from 1 hour to 5 hours to yield a metal chloride bound to a refractory oxide.

The bound catalyst precursor can be converted into the phosphate catalyst of the invention by reaction with phosphoric acid. For example, the metal chloride bound to a refractory oxide, described hereinbefore, can be heated with an excess of 85 weight percent phosphoric acid at a temperature in the range from 130°C to 200°C for a time in the range from 1 hour to 5 hours to yield the niobium or tantalum phosphate supported on a refractory oxide. Preferably, the supported phosphate catalyst is niobium phosphate on alumina, silica, or titania. More preferably, the supported phosphate catalyst is niobium phosphate on silica.

The amount of catalyst which is employed in the process of this invention is any amount which is effective in producing the desired non-cyclic polyalkylenepolyamine products. The amount of catalyst varies considerably depending upon the specific reactants and reaction conditions employed. Typically, in a batch reactor the amount of catalyst is in the range from 0.1 weight percent to 20 weight percent based on the weight of reactant amine. Preferably, the amount of catalyst is in the range from 1 weight percent to 15 weight percent based on the weight of reactant amine.

The process of this invention can be carried out in any suitable reactor, including batch reactors, continuous fixed-bed reactors, slurry reactors, fluidized bed reactors, and catalytic distillation reactors. Preferably, the reactor is a continuous fixed-bed reactor.

The reactants are contacted with the catalyst at any operable temperature which promotes the amination process of this invention and yields the desired linearly-extended polyalkylenepolyamine products. Typically, the temperature is in the range of from 200°C to 400°C. Preferably, the temperature is in the range of from 250°C to 400°C. More preferably, the temperature is in the range of from 270°C to 320°C. Below the preferred lower temperature the conversion of difunctional alcohol may be low. Above the preferred upper temperature the selectivity for non-cyclic polyalkylenepolyamines may decrease.

Likewise, the difunctional aliphatic alcohol and amine reactants are contacted with the catalyst at any operable pressure which promotes the amination process of this invention and yields the desired linearly-extended polyalkylenepolyamine products. Typically, the pressure is sufficient to maintain the reactants in the liquid state at the temperature of the reaction. Preferably, the pressure is in the range of from atmospheric to 4000 psig (28 MPa gauge). More preferably, the pressure is in the range of from 500 psig to 3000 psig (3 to 21 MPa gauge). Most preferably, the pressure in the range from 1000 psig to 2000 psig (7 to 14 MPa gauge). In batch reactors the pressure is autogenous, and depends on the vapor pressure of the reactants and products, and on the temperature of the reaction.

When the process of this invention is conducted in a continuous flow reactor, the flow rate of the reactants can be varied. Generally, the difunctional alcohol and the reactant amine are premixed to form a feed stream which is fed into the reactor at any operable flow rate which yields predominantly linearly-extended polyalkylenepolyamine products. The flow rate is expressed as the liquid hourly space velocity (LHSV) and is given in units of grams of total reactants per milliliter of total reactor volume per hour, g ml⁻¹ hr⁻¹. It is preferred to employ a liquid hourly space velocity of reactants in the range from 0.1 g ml⁻¹ hr⁻¹ to 10.0 g ml⁻¹ hr⁻¹, more preferably in the range from 0.5 g ml⁻¹ hr⁻¹ to 4.0 g ml⁻¹ hr⁻¹. It should be understood that the space velocity controls the residence time of the reactants in a continuous flow reactor.

When the process of this invention is conducted in a batch reactor, the reaction time determines the length of contact between the reactants and the catalyst. Any reaction time which yields the desired linearly-extended polyalkylenepolyamine products is acceptable. The reaction time depends on the quantity of reactants, the quantity of catalyst, the temperature of the reaction and desired degree of conversion. Preferably, the reaction time in a batch reactor is in the range from 1 hour to 20 hours.

When the difunctional aliphatic alcohol and the reactant amine are contacted in accordance with the process of this invention, a reaction occurs to form a polyalkylenepolyamine product. Specifically, the hydroxyl moiety of the difunctional alcohol reacts with the reactant amine to form the polyalkylenepolyamine product and water is eliminated as a by-product. If the difunctional alcohol contains two or more hydroxyl moieties, the reactant amine may react at each hydroxyl. Preferably, the product is a mixture of polyalkylenepolyamines enriched in linearly-extended products, such as straight-chain or branched chain compounds. For example, if the reactants are monoethanolamine and ethylenediamine, the polyalkylenepolyamine products are preferably diethylenetriamines and the straight and branched tetraethylenetetramines.

The preferred linearly-extended polyalkylenepolyamines which do not contain a piperazine moiety can be represented by the general formula V: 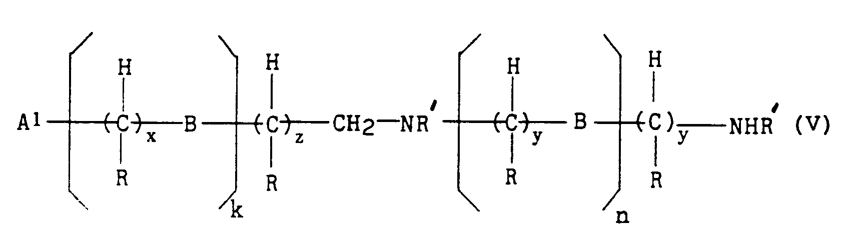
wherein each B, R and R' is as defined in connection with formula I; each x and y is independently a positive integer from 2 to 12; z is a positive integer from 1 to 12; k and n are each independently a positive integer from 0 to 150; and wherein A¹ is OH or NHR' or: 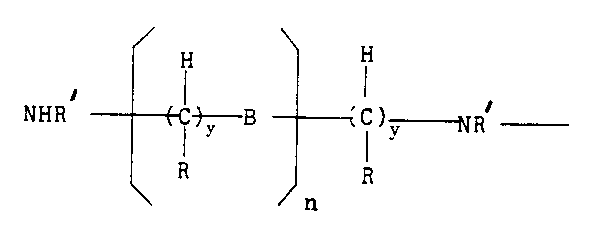
Preferably, each R and R' is hydrogen. More preferably, each R and R' is hydrogen, A¹ is NH₂, k is 0, y is 2, and z is 1. Most preferably, each R and R' is hydrogen, A¹ is NH₂, k is 0, y is 2, z is 1, and n is 1, 2, or 3; thus, the polyalkylenepolyamines are diethylenetriamine, triethylenetetramine, and tetraethylenepentamine.

The preferred alcohol-extended and amine-extended piperazine products can be represented by the general formula VI: 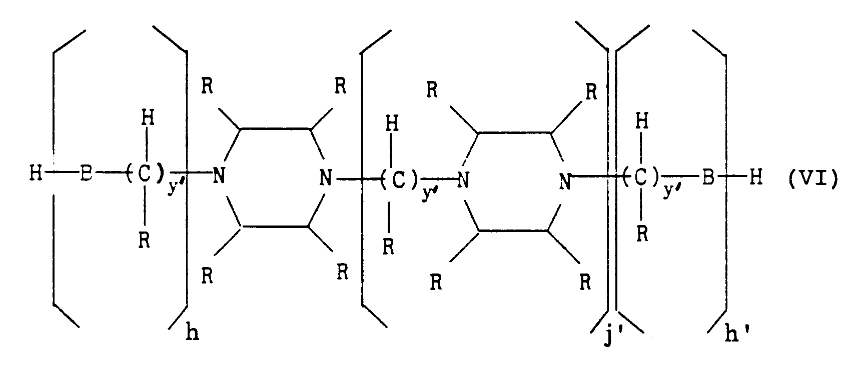
wherein each B, R and R' is as defined in connection with formula I; each y' is independently an integer from 0 to 12; h and h' are each independently integers from 0 to 6; and j' is an integer from 0 to 6. Some examples of products which satisfy this formula include N-(2-aminoethyl)piperazine, N-(2-hydroxyethyl) piperazine, 1,2-bis(piperazinyl)ethane (i.e. bispiperazine) and higher oligomers of piperazine. Preferably, each R is hydrogen. More preferably, each R and R' is hydrogen, y' is 1 or 2, j' is 1 or 2, h and h' are each independently 0-2, and each B is NR'. Most preferably, each B is NR', each R is hydrogen, y' is 2, h is 1, j' and h' are each 0, and the product is N-(2 -aminoethyl)piperazine.

For the purposes of this invention, "conversion" is defined as the weight percentage of difunctional aliphatic alcohol lost as a result of reaction. The conversion can vary widely depending upon the reactants, the form of the catalyst, and the process conditions such as temperature, pressure, and flow rate. Within the preferred temperature range, as the temperature increases the conversion generally increases. Within the preferred space velocity range, as the space velocity increases the conversion generally decreases. Typically, the conversion of the difunctional alcohol is at least 3 weight percent. Preferably, the conversion is at least 10 weight percent, more preferably at least 20 weight percent, even more preferably at least 35 weight percent, and most preferably at least 50 weight percent.

Likewise, for the purposes of this invention "selectivity" is defined as the weight percentage of converted difunctional alcohol which forms a particular polyalkylenepolyamine product. Typically, the selectivities also vary widely depending upon the reactants, the form of the catalyst, and the process conditions. Typically, the process of this invention achieves high selectivities to linearly-extended polyalkylenepolyamines. Within the preferred temperature range, as the temperature increases the selectivity for linearly-extended polyalkylenepolyamines generally decreases. Within the preferred space velocity range, as the space velocity increases the selectivity for linearly-extended polyalkylenepolyamines generally increases. Preferably, the combined selectivity to all linearly-extended polyalkylenepolyamines is at least 50 weight percent, more preferably, at least 60 weight percent, and most preferably at least 70 weight percent. Preferably, the combined selectivity to alkyl-extended, alcohol-extended and/or amine-extended piperazine is at least 40 weight percent, more preferably at least 60 weight percent, and most preferably at least 80 weight percent. In the specific amination of monoethanolamine by piperazine, the product N-(2-aminoethyl) piperazine is produced in a selectivity of at least 25 weight percent, more preferably, at least 45 weight percent and most preferably, at least 60 weight percent.

Where applicable, the efficiency of the amination reaction in forming linearly-extended polyalkylenepolyamines is measured by the weight ratio of diethylenetriamine to piperazine, abbreviated DETA/PIP. The higher the value of this ratio, the more non-cyclic (NC) polyalkylenepolyamines are present in the product mixture. Preferably, the DETA/PIP weight ratio is at least 3. More preferably, the DETA/PIP weight ratio is at least 10; most preferably, at least 20. Another measure of the efficiency of forming non-cyclic products is the weight percentage of triethylenetetramines which are non-cyclic, %NC TETA. Preferably, %NC TETA is at least 50 weight percent. More preferably, %NC TETA is at least 75 weight percent; most preferably, at least 90 weight percent.

The following examples illustrate the invention, but are not intended to be limiting thereof. All percentages are given as weight percent, unless noted otherwise. In some instances the following abbreviations are used to indicate the reactants and products:
- MEA: monoethanolamine
- EDA: ethylenediamine
- AEEA: N-(2-aminoethyl)ethanolamine
- DETA: diethylenetriamine
- TETA: triethylenetetramine
- TEPA: tetraethylenepentamine
- PIP: piperazine
- AEP: N-(2-aminoethyl)piperazine
- DABCO: 1,4-diaza-[2.2.2]-bicyclooctane
- DIAEP: N,N'-bis(2-aminoethyl)piperazine
- PEEDA: (piperazinylethyl)ethylenediamine
- BISPIP: 1,2-bis(piperazinyl)ethane or bispiperazine
- HEBIS: N-(2-hydroxyethyl)bispiperazine
- TRISPIP: N,N'-bis(2-piperazinylethyl) piperazine or trispiperazine
- HETRIS: N-(2-hydroxyethyl)trispiperazine
- EG: ethylene glycol

### Example 1

### (a) Preparation of Niobium Phosphate Catalyst

A mixture was prepared containing niobic acid, Nb₂O₅.xH₂O, (60.33 g; 0.211 moles; Niobium Products Corp., AD-460) and 85 percent phosphoric acid (602.20 g; 5.22 moles). The mixture was heated to 150°C with stirring. The niobium oxide dissolved to form a pink solution, and upon heating a precipitate forms. The precipitate was boiled in the phosphoric acid for about 2 hours. The boiled mixture was cooled to room temperature, and the liquid phase was decanted from the precipitate. The precipitate was washed by stirring with 500 ml of water, after which the aqueous mixture was filtered. The washing and filtering cycle was repeated five times. The filtered solid was dried at 110°C under air for 2 1/2 days to yield a catalyst of niobium phosphate. The elemental analysis and X-ray diffraction pattern of the catalyst were consistent with the composition NbOPO₄. The P/Nb mole ratio, as determined by neutron activation analysis and X-ray fluorescence, was 1.03. The catalyst was calcined at 300°C prior to use.

### (b) Amination in Batch Reactor

A mixture (50 g) of ethylenediamine and monoethanolamine in an EDA/MEA mole ratio of 2/1 was loaded into a 300 ml autoclave with the niobium phosphate catalyst (2.0 g), prepared hereinabove. The autoclave was sealed and purged with nitrogen gas three times. The temperature of the autoclave was raised to 265°C and held thereat for a total of 600 minutes. The cooled reaction products were analyzed by gas-phase chromatography. A CAM (Carbowax amine deactivated) capillary column (30 m x 0.25 mm dia.) was employed for the analysis of total amines. Isomer distributions were determined on an SE-54 capillary column (30 m x 0.25 mm dia.). A DB-5 (15 m x 0.25 mm dia.) column was also used for analyzing products and isomers. The conversion of MEA was 60 percent and the selectivities, based on a feed-free and water-free basis, were the following: DETA, 84.1 percent; TETA, 7.1 percent; AEEA, 5.5 percent; PIP, 1.9 percent; and AEP, 1.4 percent. The DETA/PIP weight ratio was 44.2 and the percentage of non-cyclic TETA's (%NC TETA) was 94.7 percent. The data show that monoethanolamine was aminated with ethylenediamine in the presence of an unsupported niobium phosphate catalyst to a mixture of polyethylenepolyamines which are predominantly non-cyclic.

### (c) Amination in Continuous Flow Reactor

The niobium phosphate catalyst (10 g), prepared hereinabove, was loaded into a stainless steel, tubular, fixed-bed continuous flow reactor approximately 6 inches long x 0.5 inch (153 mm x 13 mm) diameter. A feedstream comprising monoethanolamine and ethylenediamine in an EDA/MEA mole ratio of 2/1 was fed upward through the catalyst for several days at a variety of temperatures, pressures, and flow rates. The process conditions and results are presented in Table I.

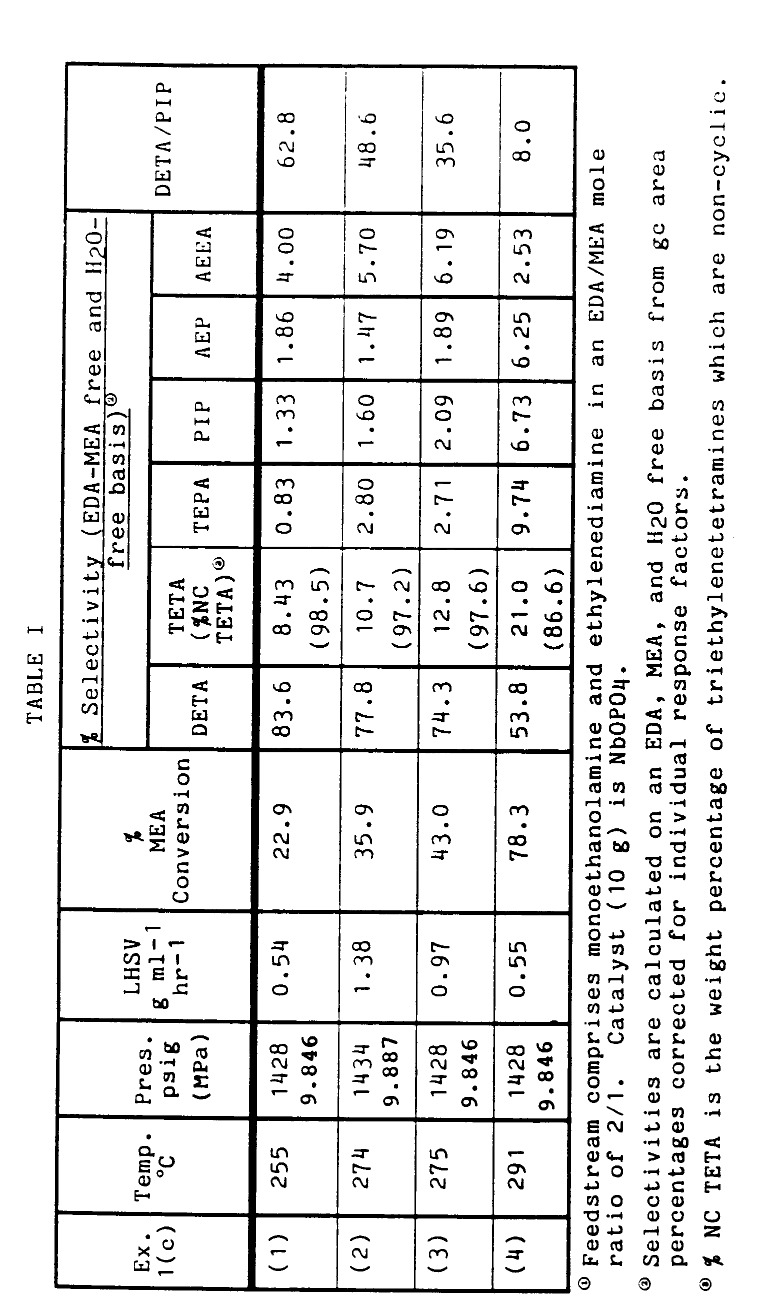

It is seen that niobium phosphate catalyzes the amination of monoethanolamine by ethylenediamine to predominantly non-cyclic polyethylenepolyamines, including diethylenetriamine and triethylenetetramines.

### Examples 2 (a-g)

### Preparation of Niobium Phosphate Catalysts

### Catalysts 2(a-d)

A series of four niobium phosphate catalysts having P/Nb mole ratios less than 1.35 was prepared generally as follows:
A predetermined amount of 85 weight percent phosphoric acid was diluted with water to a total of 30 ml volume. The aqueous phosphoric acid solution (30 ml) was poured over niobic acid (10 g; Nb₂O₅·xH₂O; Niobium Products Corp., CBMM number AD222), and the mixture was stirred at room temperature (about 23°C) for 1 hour. After stirring, the mixture was dried under air at room temperature overnight. The mixture was further dried at 130°C for 4 hours, and then dried at 300°C overnight to yield a niobium phosphate catalyst. Details of the preparations and the P/Nb mole ratios are given in Table II.

### Catalyst 2(e)

Niobic acid, Nb₂O₅·xH₂O (10 g; Niobium Products Corp., CBMM number AD222), was heated at 185°C for 2 hours in 100 g of 85 weight percent phosphoric acid. After cooling to room temperature, the material was stirred with 100 ml of water. The aqueous mixture was filtered, then washed with 200 ml of water and filtered three times more. The washed material was dried at 130°C for 4 hours and then dried overnight at 300°C to yield a niobium phosphate catalyst.

### Catalyst 2(f)

Niobic acid powder, Nb₂O₅·xH₂O (120 g; Niobium Products Corp., CBMM number AD460) and phosphoric acid (1200 g; 85 weight percent) were stirred and heated to boiling. At 145°C the niobic acid dissolved. The solution was heated further, and at 154°C a solid precipitated. Upon further heating and at about 165°C the mixture formed a paste or thick slurry. The slurry was heated at 165°C for 1 hour and then cooled to room temperature overnight. To the cooled mixture was added 500 cc of water. The aqueous mixture was filtered, then washed with 500 cc of water and filtered three more times. The washed material was dried at 120°C overnight, then calcined at 300°C overnight to yield a niobium phosphate catalyst.
The catalysts 2(a-f) were analyzed by neutron activation analysis and X-ray fluorescence to determine the P/Nb mole ratio. It was found that the P/Nb mole ratios varied from 0.28 to 1.35, as shown in Table II.

### (g) Amination of Monoethanolamine

Each of the niobium phosphate catalysts 2(a-f), prepared hereinabove, was tested in the amination of monoethanolamine by ethylenediamine according to the following general procedure:
A mixture (45.0 g) comprising monoethanolamine and ethylenediamine in an EDA/MEA mole ratio of 2/1 was loaded into a 300 ml Parr pressure reactor equipped with mechanical agitation. The niobium phosphate catalyst (1 g) to be tested was added to the reactor, and the reactor was sealed. The sealed reactor was flushed three times with nitrogen gas. After flushing, the temperature of the reactor was raised to 290°C over a period of 1 hour. The reaction mixture was held with stirring at 290°C for either 300 minutes or 600 minutes. The reaction mixture was then cooled to room temperature. Samples of the used catalysts at 300 minutes and 600 minutes were analyzed by neutron activation analysis and X-ray flourescence with the results shown in Table II.

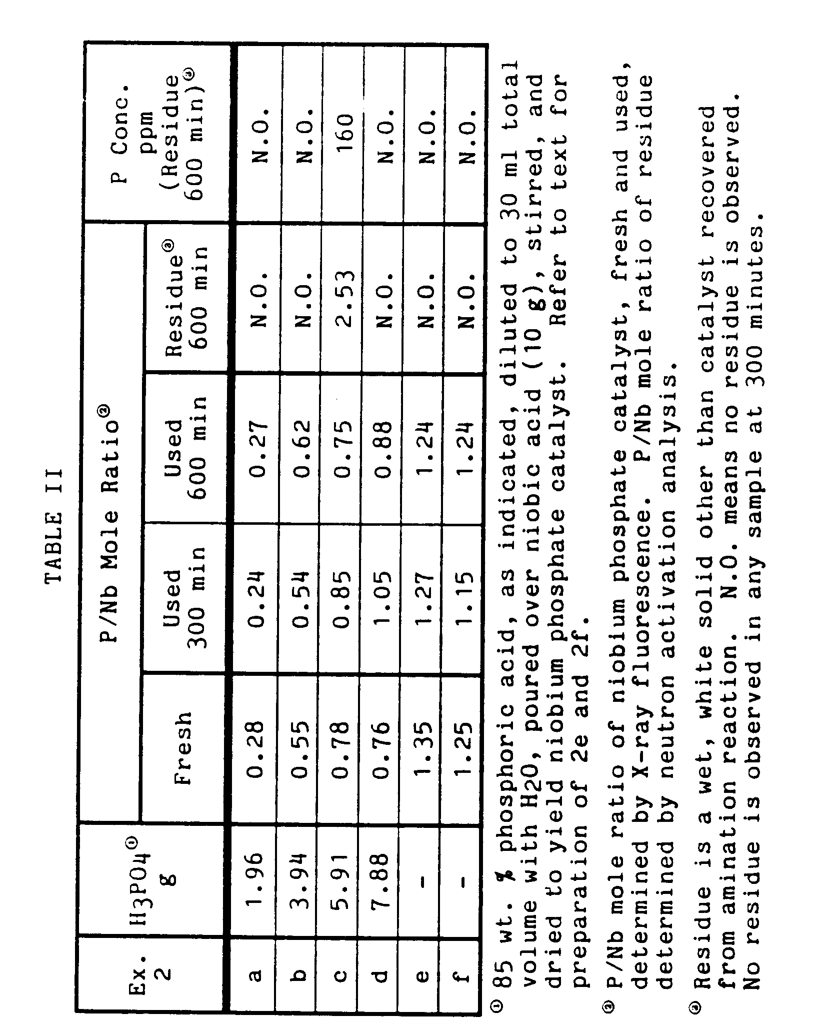

It is seen that the fresh niobium phosphate catalysts prepared hereinabove have a P/Nb mole ratio in the range of from 0.28 to 1.35. It is further seen that the P/Nb mole ratio in the used catalysts is close to the mole ratio of the fresh catalysts. This result indicates that the niobium phosphate catalysts substantially maintain their compositional integrity throughout the amination reaction. With the exception of catalyst 2(c), all examples 2(a, b, d-f) showed no residue at either 300 minutes or 600 minutes. In one sample (2c) a white, fluffy residue was separated wet from the catalyst at 600 minutes, and found to have a P/Nb mole ratio of 2.53. This residue indicates that some decomposition of catalyst 2(c) occurred producing a material which was significantly enriched in phosphorus.

In addition to the analysis of the used catalysts, an analysis of the products of the amination reaction was conducted by gas phase chromatography on an SE 54 capillary column, as described in Example 1(b) hereinbefore. The products for each run were found to be predominantly linearly-extended polyethylenepolyamines, including diethylenetriamine and linear and branched triethylenetetramines, as shown in Table III for Examples 2(a) and 2(f).

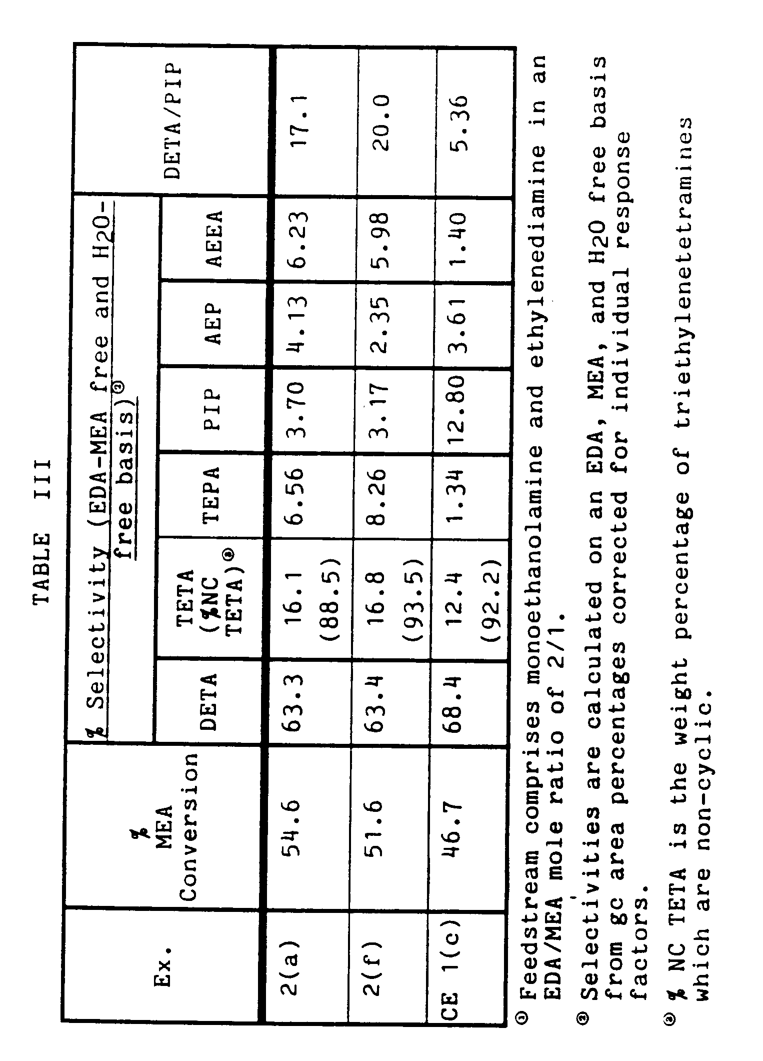

### Comparative Example 1

A series of four niobium phosphate materials with P/Nb mole ratios greater than 1.35 was prepared as described hereinbelow. The comparative materials CE 1(a-d) were analyzed by neutron activation analysis and X-ray fluorescence to determine their P/Nb mole ratio, which is given in Table IV.

### Comparative Material CE 1(a)

Phosphoric acid (11.82 g; 85 weight percent) was diluted to 30 ml total volume with water. The aqueous phosphoric acid solution was poured over niobic acid (10.0 g; Niobium Products Corp., CBMM number AD222), and the mixture was stirred and dried according to the general procedure of Examples 2(a-d) to yield a niobium phosphate having a P/Nb mole ratio of 1.41.

### Comparative Material CE 1(b)

Pyrophosphoric acid, H₄P₂O₇ (210 g), was melted at about 70°C and poured over niobic acid (19.4 g). The resulting mixture was heated to a temperature of 210°C whereupon the niobic acid dissolved. Heating was continued to 230°C whereupon a precipitate formed. Heating was continued further until a temperature of 270°C was obtained, and the temperature was held thereat for 15 minutes. The heated mixture was cooled to room temperature. The cooled mixture was washed with 200 ml of water and filtered. The washed material was dried at 150°C for 1 hour and at 300°C overnight to yield a niobium pyrophosphate having a P/Nb mole ratio of 1.74.

### Comparative Materials CE 1(c)

Phosphoric acid (15.76 g; 85 wt. %) was diluted to 30 ml total volume with water. The aqueous phosphoric acid solution was poured over niobic acid (10.0 g; Niobium Products Corp., CBMM number AD222), and the mixture was stirred and dried according to the general procedure of Examples 2(a-d) to yield a niobium phosphate having a P/Nb mole ratio of 2.22.

### Comparative Material CE 1(d)

A niobium phosphate having a P/Nb mole ratio of 3.74 was prepared as in Comparative Experiment CE 1(c) hereinabove, except that the amount of 85 weight percent phosphoric acid was 23.64 g.

### (e) Amination of Monoethanolamine

Each of the niobium phosphate comparative materials CE 1(a-d), prepared hereinabove, was tested in the amination of monoethanolamine by ethylenediamine according to the general procedure of Example 2(g). Samples of the used comparative materials at 300 minutes and 600 minutes were analyzed by neutron activation analysis and X-ray fluorescence, as shown in Table IV.

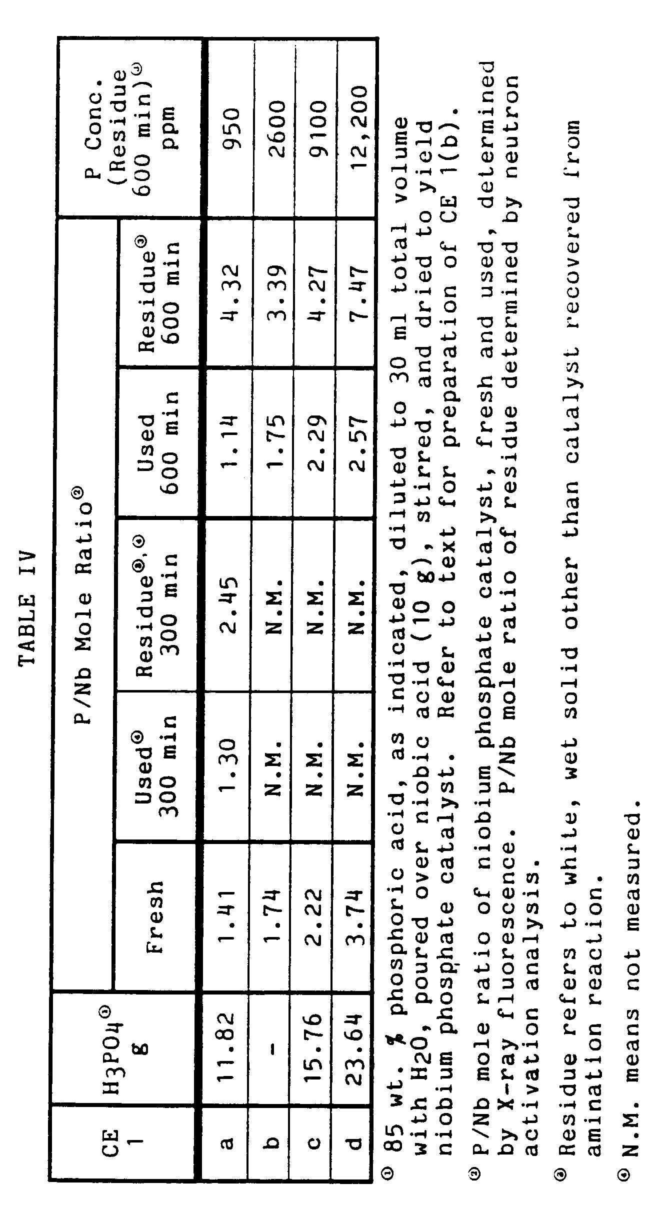

It is seen that the used comparative materials CE 1(a) and CE 1(d) exhibit a substantially lower P/Nb mole ratio than the corresponding fresh materials. In addition, in each comparative example a white, wet residue is obtained which has a P/Nb mole ratio at least double the ratio of the fresh catalyst. This residue is a material which comes from the catalyst and which is enriched in phosphorus content. The concentration of phosphorus in this material is observed to increase significantly as the P/Nb mole ratio of the fresh catalyst increases. It is concluded that the comparative materials are disintegrating in the amination reaction.

When the concentration of phosphorus in the residues (600 minutes) of the Comparative Examples is compared with the residue (600 minutes) of Example 2(c), it is seen that the residues of the Comparative Examples exhibit at least 6 to 76 times greater phosphorus concentration than the residue of Example 2(c). Moreover, when Comparative Examples CE 1(a-d) are compared with Examples 2(a-f) it is seen that fresh catalysts having a P/Nb mole ratio less than 1.35 retain their physical integrity in the amination reaction for a longer period of time than do fresh comparative materials having a P/Nb mole ratio greater than 1.4.

In addition to the analyses described hereinabove, the products of the amination reaction were analyzed by gas phase chromatography for each comparative experiment. The reaction products were found to be predominantly linearly-extended and non-cyclic polyethylenepolyamines, including diethylenetriamine and linear and branched triethylenetetramines. However, when Comparative Experiment CE 1(c) is compared with Examples 2(a) and 2(f) as shown in Table III (DETA/PIP ratio), it is observed that the weight ratio of linearly-extended non-cyclic products to cyclic products of the comparative material (P/Nb mole ratio, 2.22) is significantly lower than the weight ratio of linearly-extended non-cyclic to cyclic products of the Examples (P/Nb mole ratio, 0.28 and 1.25).

### Example 3

### Amination of Ethylene Glycol

Piperazine (51.6 g; 0.60 moles), ethylene glycol (12.4 g; 0.20 moles), and the catalyst of Example 1(a) (1.0 g) were loaded into a 300 ml, stirred autoclave. The reactor was sealed and flushed with nitrogen. The contents were heated for 4 hours at 300°C. Upon cooling to room temperature the products were analyzed by gas chromatography with the following results: conversion of EG, 98 percent; selectivities to HEP, 11.4 percent; BISPIP, 52.5 percent; DABCO, 5.2 percent; HEBIS, 6.2 percent; TRISPIP, 15 percent; HETRIS, 1.4 percent; and higher oligomers, 8.3 percent. It is seen that the amination products are predominantly linearly extended piperazines. Moreover, the quantity of undesirable internally cyclized products which was formed, such as DABCO, is low.

### Example 4 Amination of Hydroxyethylpiperazine

Piperazine, hydroxyethylpiperazine, solvent, and the catalyst of Example 1(a) were loaded into a 300 ml stirred autoclave in the proportions noted in Table V. The reactor was sealed and flushed with nitrogen, and the contents were heated as designated in Table V. Upon cooling to room temperature the product mixture was analyzed by gas chromatography with the results shown in Table V.

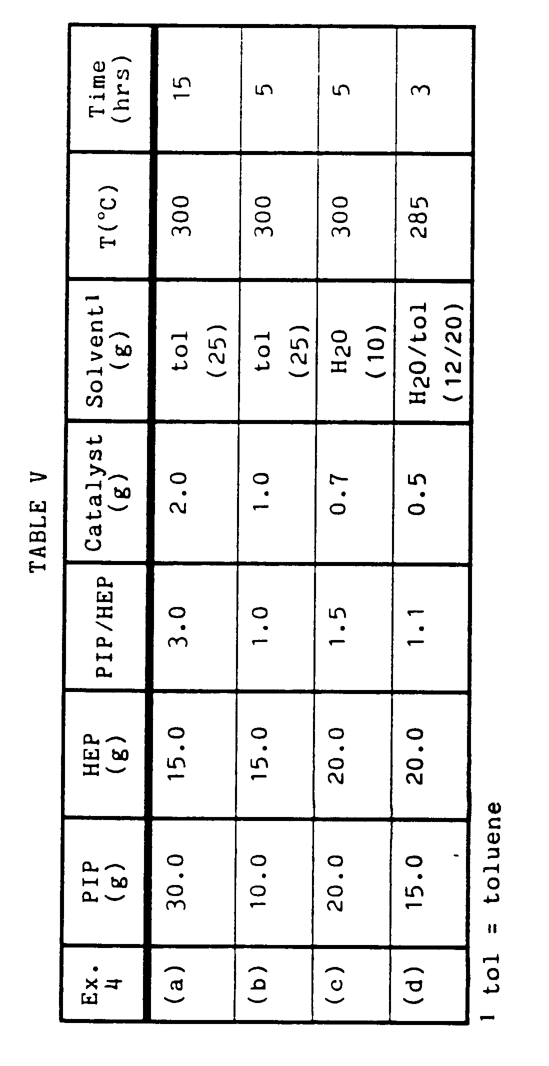

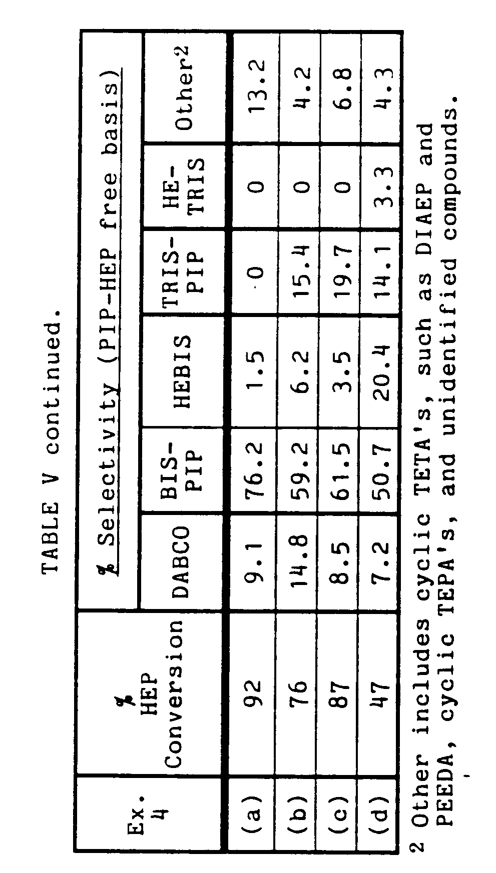

The data show that hydroxylethylpiperazine is aminated by piperazine in the presence of a catalyst of niobium phosphate to predominantly bispiperazine, which is an amine-extended piperazine. Moreover, the quantity of undesirable products, such as DABCO, is low.

### Example 5 Amination of Monoethanolamine

Four samples of the catalyst of Example 1(a) (Niobium Phosphate Catalyst) were calcined prior to use at the following temperatures: (a) 300°C, (b) 700°C, and (c) 1000°C. Each catalyst was employed in an amination reaction according to the following general procedure: Monoethanolamine (20.3 g; 0.33 moles), piperazine (28.7 g; 0.34 moles), and catalyst (1.0 g) were loaded into a 300 ml stirred batch reactor. The reactor was flushed with nitrogen three times, then heated to 300°C. The reaction mixture was maintained at 300°C for five hours, then cooled to room temperature and analyzed by gas phase chromatography. A CAM (Carbowax amine deactivated) capillary column (30 m x 0.25 mm dia.) was employed to measure total amine products. Isomer distributions were measured on an SE-30 capillary column (30 m x 0.25 mm dia.). An SE-54 capillary column (30 m x 0.25 mm dia.) was also used in analyzing for total amine content and isomer distribution. The results are presented in Table VI.

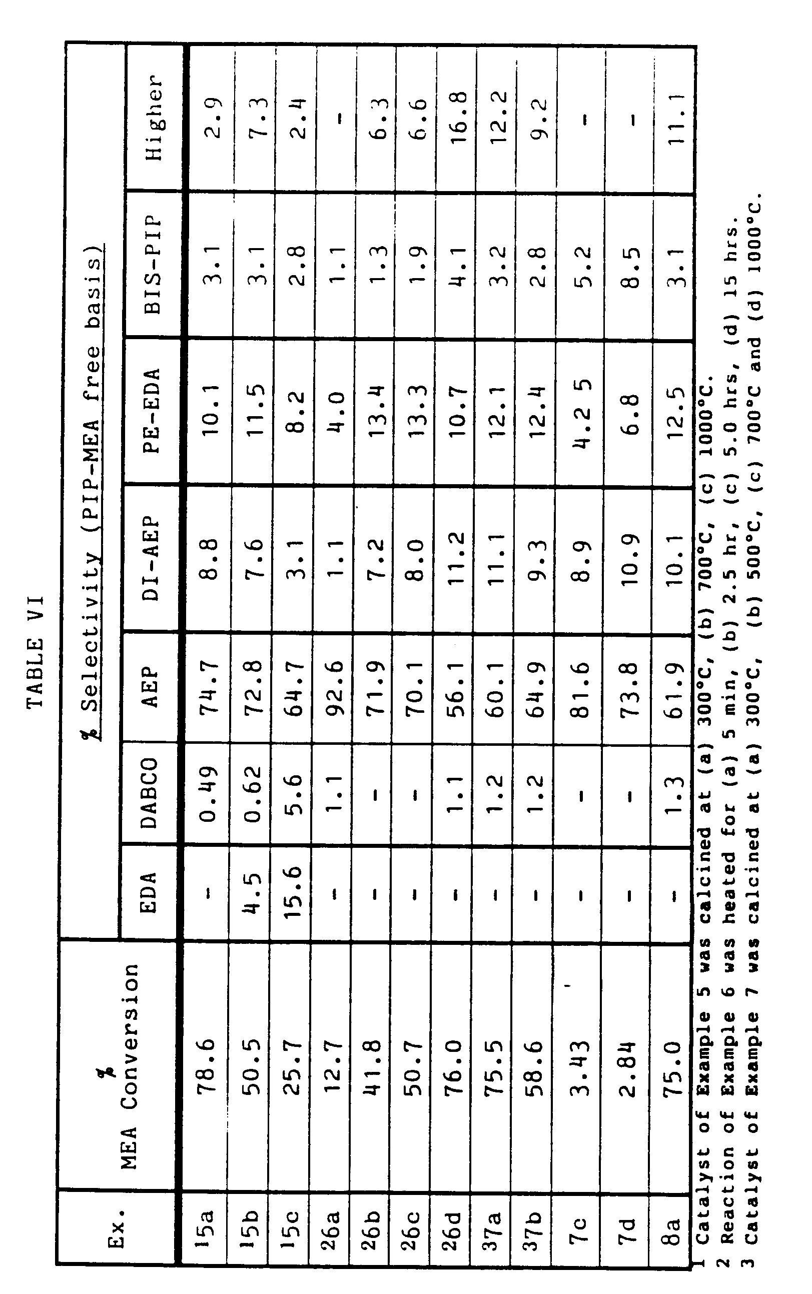

It is seen that monoethanolamine is aminated with piperazine in the presence of a niobium phosphate catalyst to predominantly aminoethylpiperazine. The conversion of monoethanolamine is greatest when the catalyst is calcined at 300°C prior to use. In addition, the quantity of undesirable products, such as DABCO, is low.

### Example 6

### (a) Preparation of Niobium Phosphate Catalyst

Silica (Shell Silica balls, 15.10 g) was dried at 300°C overnight, then placed in a flask with niobium chloride, NbCl₅, (4.25 g, 15.7 mmoles). Acetonitrile was added to the flask with stirring in an amount sufficient to cover the balls and dissolve the niobium chloride. Thereafter, the acetonitrile was removed by rotary evaporation. Throughout the evaporation process the flask was rotated in order to coat the balls evenly with niobium chloride. The coated balls were heated at 50°C for about 2 hours in vacuo, then cooled to room temperature to yield a composition of niobium chloride on silica. This composition was immediately added to 150 ml of 85 percent phosphoric acid at 160°C. The resulting mixture was heated to 195°C and maintained thereat for 5 minutes. The heated mixture was cooled to room temperature, whereupon 400 ml of water were added. The resulting aqueous mixture was stirred and filtered. The filtered solid was washed with water and refiltered. The washing and filtering procedures were repeated twice more. The washed solid was dried at 150°C for 3 hours, and then calcined under air at 300°C overnight to yield a catalyst of niobium phosphate bound to silica.

### (b) Amination of Monoethanolamine

Monoethanolamine (20.5 g; 0.34 moles), piperazine (29.5 g; 0.34 moles), and the silica-supported niobium phosphate catalyst (1.0 g), prepared hereinabove, were loaded into a 300 ml stirred batch reactor. The reactor was flushed with nitrogen three times, then heated to 300°C. The reaction was maintained at 300°C for the following times: (a) 5 minutes, (b) 2.5 hours, (c) 5 hours, (d) 15 hours, then cooled to room temperature and analyzed. The results are presented in Table VI. It is seen that mono-ethanolamine is aminated with piperazine in the presence of a silica-supported niobium phosphate catalyst to predominantly aminoethylpiperazine; whereas, only low amounts of undesirable products, such as DABCO, are formed.

### Example 7 (Comparative) Amination of Monoethanolamine

Niobic acid, Nb₂O₅·xH₂O (Niobium Products Corp., CBMM number AD222) was pressed at a pressure of 20,000 psi (140 MPa) into cylindrical pellets 1 inch (25mm) in diameter by 1 inch (25mm) in height. The pellets each contained approximately 25 g of niobic acid. The pressed pellets were dried at 120°C for 4 hours, then slowly heated under air to a calcination temperature of (a) 300°C, (b) 500°C, (c) 700°C; or (d) 1000°C and held overnight. The calcined pellets were cooled, crushed, and sieved to 14-20 mesh size (Tyler equivalent designation) (1.18mm-850»m). The catalysts were employed in the amination of monoethanolamine by piperazine, as in Example 5, with the results shown in Table VI. It is seen that monoethanolamine is aminated with piperazine in the presence of a catalyst of niobium oxide to predominantly aminoethylpiperazine and other amine-extended piperazines. The yield of undesirable products, such as DABCO, was low. It is also seen that when calcined to a temperature greater than 500°C, the niobic acid or niobium oxide catalyst gave very little activity whereas the niobium phosphate catalyst gave excellent activity.

### Example 8 (Comparative) Amination of Monoethanolamine

Niobic acid, Nb₂O₅·xH₂O (Niobium Products Corp., CBMM number AD460) was pressed at 20,000 psig (140 MPa gauge) into cylindrical pellets 1 inch (25 mm) in diameter by 1 inch (25mm) in height. The pressed pellets were dried at 120°C for 4 hours, then heated slowly under air to a calcination temperature of 300°C and held overnight. The calcined pellets were crushed and sieved to 14-20 mesh size (Tyler equivalent designation) (1.18mm-850»m) prior to use in the reactor. The catalyst was employed in Example 8(a) in the amination of monoethanolamine by piperazine, as in Example 5, with the results shown in Table VI. The data show that monoethanolamine is aminated by piperazine in the presence of niobic acid to predominantly aminoethylpiperazine. The results are comparable to those of Example 7(a). The results also show that when calcined at 300°C, the selectivity of the niobic acid catalyst to AEP is less than that of the niobium phosphate catalyst.

### Example 9

### (a) Preparation of Tantalum Phosphate Catalyst

TaCl₅ (100.0 g, 0.28 moles) was added to 600 g of 85 percent phosphoric acid with stirring. The mixture was heated to 185°C, maintained thereat for 1 hour, and then cooled overnight. After cooling, 500 ml of water were added to the mixture with stirring, and the mixture was filtered. The washing and filtering steps were repeated three times. The filtered solid was dried overnight at 120°C, then calcined at 300°C under air over a second night to yield a tantalum phosphate catalyst.

### (b) Amination of Monoethanolamine with Piperazine

Monoethanolamine (20.3 g; 0.33 moles), piperazine (28.7 g; 0.34 moles), and the tantalum phosphate catalyst (1.0 g), prepared hereinabove, were loaded into a 300 ml stirred batch reactor. The reactor was flushed with nitrogen three times, then heated to 300°C. The reaction mixture was maintained at 300°C for five hours, then cooled to room temperature and analyzed with the following results: conversion of MEA, 11 percent; selectivities to AEP, 77.6 percent; DIAEP, 3.5 percent; PEEDA, 11.4 percent; BISPIP, 7.5 percent. There is no detectable DABCO or higher oligomers. The data show that monoethanolamine is aminated with piperazine in the presence of a tantalum phosphate catalyst to predominantly amine-extended piperazines.

### Example 10

### (a) Preparation of Niobium Silico-phosphate Catalyst

A first mixture containing fumed silica (10.0 g; Cabosil M5) and 300.0 g of 85 percent phosphoric acid was prepared and heated to 165°C. A second mixture containing niobic acid (60.26 g; Niobium Products Inc.) and 600.0 g of 85 percent phosphoric acid was prepared and heated to 145°C. When all of the niobic acid had dissolved in the phosphoric acid, the first mixture was added to the second mixture. The combined mixtures were heated to 176°C whereupon a precipitate formed. The precipitate and mother liquor were heated to 200°C, and then cooled to room temperature. The cooled mixture was diluted with 500 ml of water with stirring, and then filtered. The filtered precipitate was washed with 500 ml of water and filtered, three times each. The washed precipitate was dried overnight at 120°C and then dried a second night at 300°C to yield a niobium silico-phosphate catalyst.

### (b) Amination of Monoethanolamine with Piperazine

The amination of monoethanolamine with piperazine was conducted as in Example 5, except that the catalyst was the niobium silico-phosphate, prepared hereinabove. The results are the following: conversion of MEA, 67.9 percent; selectivities to DABCO, 1.04 percent; AEP, 65.5 percent; DIAEP, 10.1 percent; PEEDA, 10.9 percent; BISPIP, 3.9 percent; and higher oligomers, 8.6 percent. The data show that monoethanolamine is aminated with piperazine in the presence of a niobium silico-phosphate catalyst to predominantly amine-extended piperazines.

### Example 11

This example shows that a niobium phosphate catalyst has a higher selectivity to non-cyclic ethylene amines than a niobic acid catalyst.

### Amination of Monoethanolamine

Niobium phosphate catalysts 11(c) was tested in the amination of monoethanolamine by ethylenediamine according to the following general procedure:

A mixture (45.0 g) comprising monoethanolamine and ethylenediamine in an EDA/MEA mole ratio of 2/1 was loaded into a 300 ml Parr pressure reactor equipped with mechanical agitation means. The niobium phosphate catalyst (1 gram) to be tested was added to the reactor, and the reactor was sealed. The sealed reactor was flushed three times with nitrogen gas. After flushing, the temperature of the reactor was raised to 290°C over a period of 1 hour. The reaction mixture was held with stirring at 290°C for 600 minutes. The reaction mixture was then cooled to room temperature. The results are shown in Table VII.
Two different niobic acid catalysts 11(a) and 11(b) were also tested in the amination of monoethanolamine by ethylenediamine following the above procedure. The results are shown in Table VII. At nearly identical conversion, the DETA/PIP ratio and the percent non-cyclic in TETA and TEPA for the niobium phosphate catalyst are higher than those for the niobic acid catalysts.

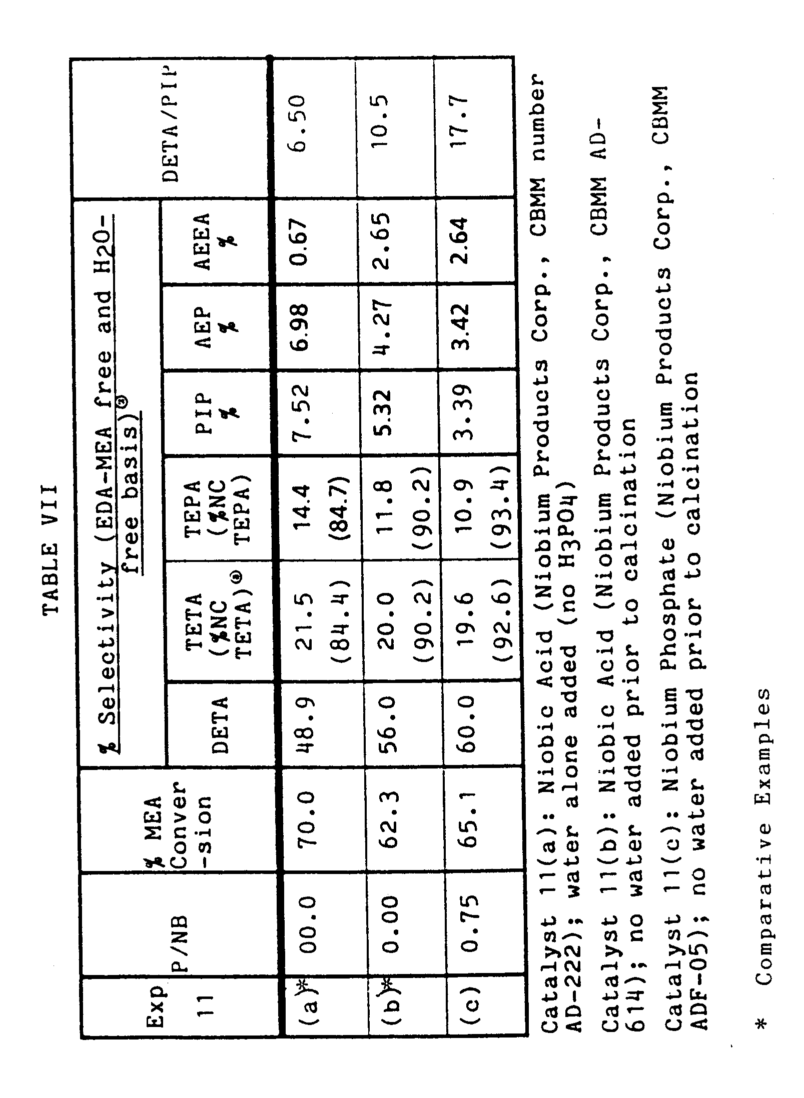

## Claims

1. A process for preparing linearly-extended polyalkylenepolyamines comprising contacting a difunctional aliphatic alcohol and a reactant amine in the presence of a catalyst under reaction conditions such that a mixture of polyalkylenepolyamines enriched in linearly-extended products is formed, characterised in that the catalyst contains a niobium phosphate or tantalum phosphate wherein the mole ratio of phosphorus to niobium or tantalum is no greater than 1.35.

2. A process as claimed in Claim 1, wherein the difunctional aliphatic alcohol is represented by the formula: 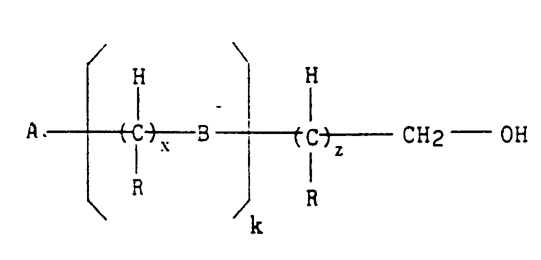 wherein A is OH or NHR'; each B is independently NR' or O; each R is independently hydrogen, hydroxyl, amino, an alkyl moiety of 1-12 carbon atoms, a hydroxyalkyl or aminoalkyl moiety of 1-12 carbon atoms, or a monocyclic aromatic moiety; each R' is independently hydrogen, hydroxyl, an alkyl moiety of 1-12 carbon atoms, a hydroxyalkyl or aminoalkyl moiety of 1-12 carbon atoms, or a monocyclic aromatic moiety; x is an integer from 2 to 12; k is 0 or an integer from 1 to 150; and z is 0 or an integer from 1 to 12.

3. A process as claimed in Claim 1, wherein the aliphatic alcohol is represented by the formula: 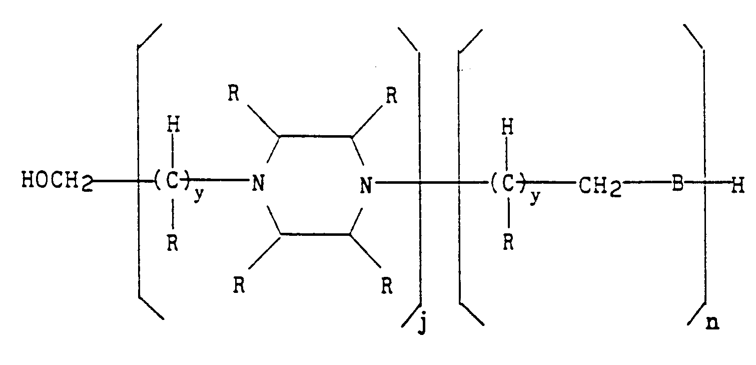 wherein B, R and R' are as defined in Claim 2; each y is independently 0 or an integer from 1 to 12; j is an integer from 1 to 6; and n is 0 or an integer from 1 to 6.

4. A process as claimed in any one of the preceding claims, wherein the reactant amine is an alkylenepolyamine represented by the formula: 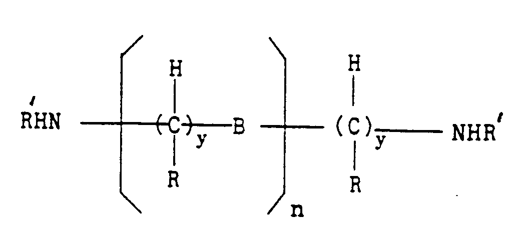 wherein B, R and R' are as defined in Claim 2; each y is independently an integer from 2 to 12; and n is 0 or an integer from 1 to 150.

5. A process as claimed in any one of Claims 1 to 3, wherein the reactant amine is represented by the formula: 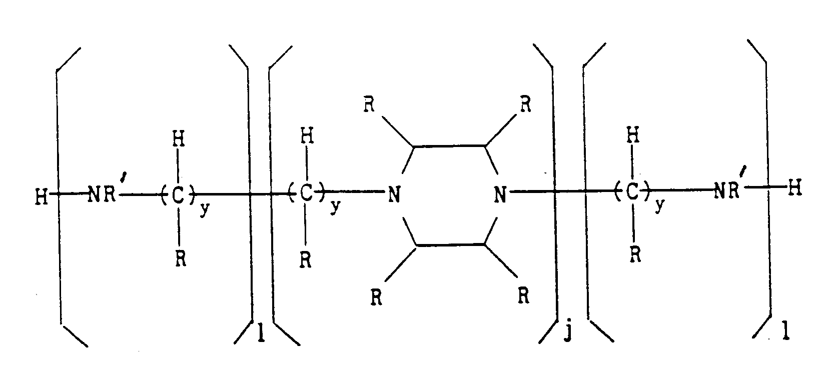 wherein B, R and R' are as defined in Claim 2; each y is independently 0 or an integer from 1 to 12; each l is independently 0 or an integer from 1 to 6; and j is an integer from 1 to 6.

6. A process as claimed in any one of Claims 3 to 5, wherein the catalyst contains a niobium phosphate or supported niobium phosphate.

7. A process as claimed in Claim 6, wherein the niobium phosphate is NbOPO₄ or hydrated NbOPO₄.

8. A process as claimed in any one of the preceding claims, wherein the said phosphate is deposited on or bound to an insoluble support.

9. A process as claimed in Claim 8, wherein the support is an alumina or hydrated alumina.

10. A process as claimed in Claim 1, wherein the difunctional aliphatic alcohol is hydroxyethylpiperazine and the reactant amine is piperazine whereby 1,2-bis(piperazinyl)ethane is prepared.

11. A process as claimed in Claim 1, wherein the difunctional aliphatic alcohol is monoethanolamine and the reactant amine is ethylenediamine whereby non-cyclic polyethylenepolyamines are prepared.

## Patentansprüche

1. Verfahren zur Herstellung linear-verlängerter Polyalkylenpolyamine umfassend
Inkontaktbringen eines difunktionellen aliphatischen Alkohols mit einem Amin-Reaktanten in Anwesenheit eines Katalysators unter derartigen Reaktionsbedingungen, daß ein Gemisch aus Polyalkylenpolyamin angereicherten, linear-verlängerten Produkten gebildet wird, dadurch gekennzeichnet, daß der Katalysator ein Niobphosphat oder Tantalphosphat enthält, worin das Molverhältnis von Phosphor zu Niob oder Tantal nicht größer als 1,35 ist.

2. Verfahren nach Anspruch 1, wobei der difunktionelle aliphatische Alkohol durch die folgende Formel dargestellt ist: 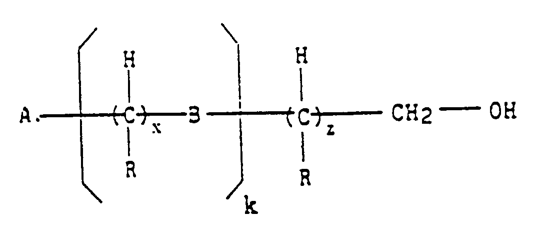 worin A OH oder NHR' ist, B unabhängig jeweils NR' oder O ist, R unabhängig jeweils Wasserstoff, Hydroxy, Amino, ein Alkylrest mit 1-12 Kohlenstoffatomen, ein Hydroxyalkyl oder Aminoalkylrest mit 1-12 Kohlenstoffatomen, oder ein monozyklischer aromatischer Rest ist, R' unabhängig jeweils Wasserstoff, Hydroxy, ein Alkylrest mit 1-12 Kohlenstoffatomen, ein Hydroxyalkyl- oder Aminoalkylrest mit 1-12 Kohlenstoffatomen oder ein monozyklischer aromatischer Rest ist, x eine ganze Zahl von 2 bis 12 ist, k 0 oder eine ganze Zahl von 1 bis 150 ist und z 0 oder eine ganze Zahl von 1 bis 12 ist.

3. Verfahren nach Anspruch 1, wobei der aliphatische Alkohol durch die folgende Formel dargestellt ist: 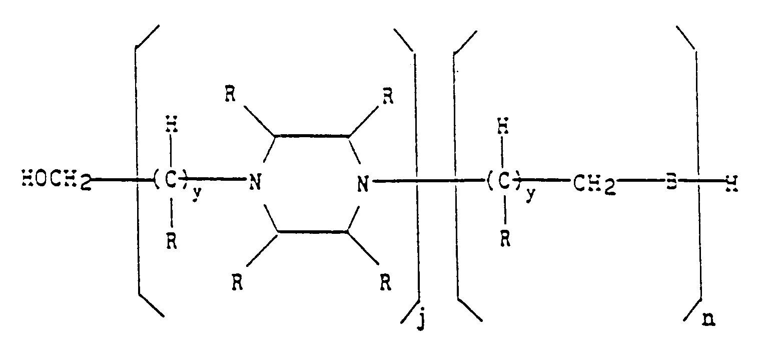 worin B, R und R' jeweils wie in Anspruch 2 definiert sind, y unabhängig jeweils 0 oder eine ganze Zahl von 1 bis 12 ist, j eine ganze Zahl von 1 bis 6 ist und n 0 oder eine ganze Zahl von 1 bsi 6 ist.

4. Verfahren nach einem der vorhergehenden Ansprüche, wobei der Amin-Reaktant ein durch die folgende Formel dargestelltes Alkylenpolyamin ist: 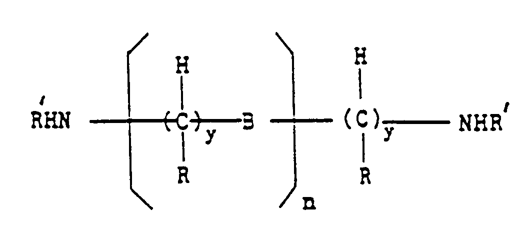 worin B, R und R' wie in Anspruch 2 definiert sind, y unabhängig jeweils eine ganze Zahl von 2 bis 12 ist und n 0 oder eine ganze Zanhl von 1 bis 150 ist.

5. Verfahren nach einem der Anspüche 1 bis 3, wobei der Amin-Reaktant durch die folgende Formel dargestellt ist: 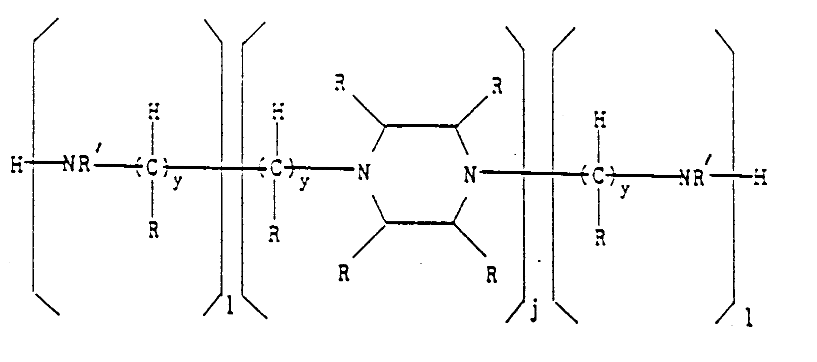 worin B, R und R' wie in Anspruch 2 definert sind, y unabhängig jeweils 0 oder eine ganze Zahl von 1 bis 12 ist, l unabhängig jeweils 0 oder eine ganze Zahl von 1 bis 6 ist und j eine ganze Zahl von 1 bis 6 ist.

6. Verfahren nach einem der Ansprüche 3 bis 5, wobei der Katalysator Niobphosphat oder unterstütztes Niobphosphat enthält.

7. Verfahren nach Anspruch 6, wobei das Niobphosphat NbOPO₄ oder hydratisiertes NbOPO₄ ist.

8. Verfahren nach einem der vorhergehenden Ansprüch, wobei das Phosphat auf einem unlöslichen Träger abgelagert oder an diesen gebunden ist.

9. Verfahren nach Anspruch 8, wobei der Träger ein Aluminiumoxid oder hydratisiertes Aluminiumoxid ist.

10. Verfahren nach Anspruch 1, wobei der difunktionelle aliphatische Alkohol Hydroxyethylpiperazin ist und der Amin-Reaktant Piperazin ist wobei 1,2-Bis(piperazinyl)ethan hergestellt wird.

11. Verfahren nach Anspruch 1, wobei der difunktionelle aliphatische Alkohol Monoethanolamin ist und der Amin-Reaktant Ethylendiamin ist, wobei nicht-zyklische Polyethylenpolyamine hergestellt werden.

## Revendications

1. Procédé de préparation de polyalkylènepolyamines étendues linéairement comprenant la mise en contact d'un alcool aliphatique difonctionnel et d'un réactif aminé en présence d'un catalyseur dans des conditions de réaction telles qu'un mélange de polyalkylènepolyamines enrichies en produits étendus linéairement se forme, caractérisé par le fait que le catalyseur contient un phosphate de niobium ou un phosphate de tantale dans lequel le rapport molaire du phosphore au niobium ou au tantale ne dépasse pas 1,35.

2. Procédé selon la revendication 1, dans lequel l'alcool aliphatique difonctionnel a pour formule : 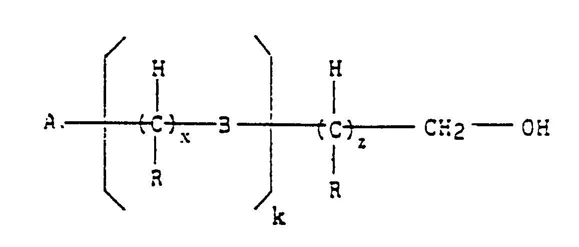 dans laquelle A est OH ou NHR' ; chaque B est indépendamment NR' ou O ; chaque R est indépendamment l'hydrogène, un hydroxyle, un amino, un radical alkyle ayant 1-12 atomes de carbone, un radical hydroxyalkyle ou aminoalkyle ayant 1-12 atomes de carbone, ou un radical aromatique monocyclique ; chaque R' est indépendamment l'hydrogène, un hydroxyle, un radical alkyle ayant 1-12 atomes de carbone, un radical hydroxyalkyle ou aminoalkyle ayant 1-12 atomes de carbone, ou un radical aromatique monocyclique ; x est un entier compris entre 2 et 12 ; k est 0 ou un entier compris entre 1 et 150 ; et z est 0 ou un entier compris entre 1 et 12.

3. Procédé selon la revendication 1, dans lequel l'alcool aliphatique est représenté par la formule : 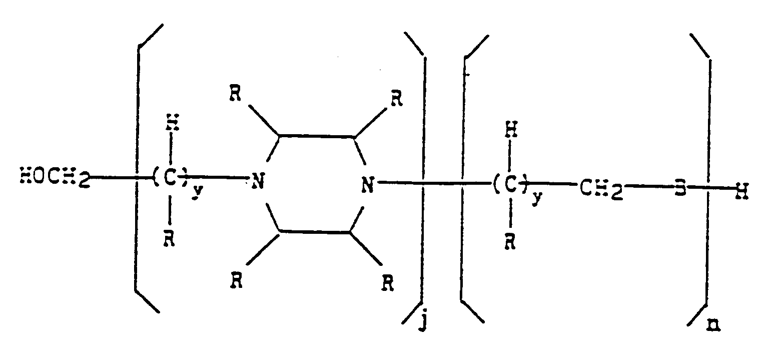 dans laquelle B, R et R' ont la définition donnée dans la revendication 2 ; chaque y est indépendamment 0 ou un entier compris entre 1 et 12 ; j est un entier compris entre 1 et 6 ; et n est 0 ou un entier compris entre 1 et 6.

4. Procédé selon l'une quelconque des revendications précédentes, dans lequel le réactif aminé est une alkylènepolyamine représentée par la formule : 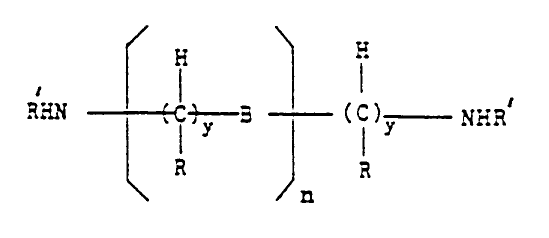 dans laquelle B, R et R' ont la définition donnée dans la revendication 2 ; chaque y est indépendamment un entier compris entre 2 et 12 ; n est 0 ou un entier compris entre 1 et 150.

5. Procédé selon l'une quelconque des revendications 1 à 3, dans lequel le réactif aminé est représenté par la formule : 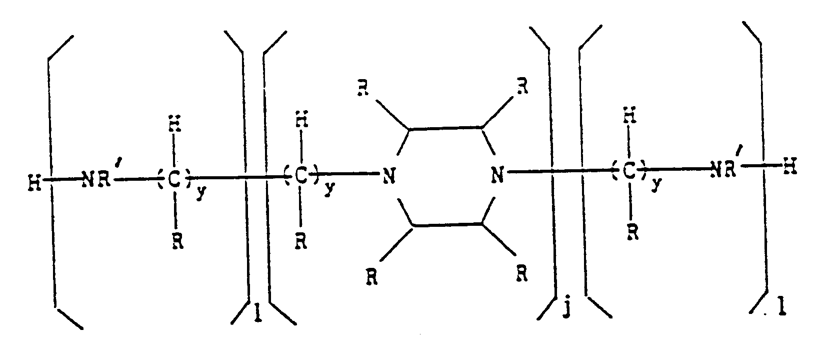 dans laquelle B, R et R' ont la définition donnée dans la revendication 2 ; chaque y est indépendamment 0 ou un entier compris entre 1 et 12 ; chaque l est indépendamment 0 ou un entier compris entre 1 et 6 ; et j est un entier compris entre 1 et 6.

6. Procédé selon l'une quelconque des revendications 3 à 5, dans lequel le catalyseur contient du phosphate de niobium ou du phosphate de niobium sur support.

7. Procédé selon la revendication 6, dans lequel le phosphate de niobium est NbOPO₄ ou NbOPO₄ hydraté.

8. Procédé selon l'une quelconque des revendications précédentes, dans lequel ledit phosphate est déposé sur un support insoluble ou lié à celui-ci.

9. Procédé selon la revendication 8, dans lequel le support est de l'alumine ou de l'alumine hydratée.

10. Procédé selon la revendication 1, dans lequel l'alcool aliphatique difonctionnel est l'hydroxyéthylpipérazine et le réactif aminé est la pipérazine, le 1,2-bis(pipérazinyl)éthane étant préparé.

11. Procédé selon la revendication 1, dans lequel l'alcool aliphatique difonctionnel est la monoéthanolamine et le réactif aminé est l'éthylènediamine, des polyéthylènepolyamines non-cycliques étant préparées.
